# EUROPÄISCHE PATENTSCHRIFT

(11) **EP 3 325 960 B1**
(45) Veröffentlichungstag und Bekanntmachung des Hinweises auf die Patenterteilung: **01.11.2023**
(21) Anmeldenummer: 16734578.4
(22) Anmeldetag: 16.06.2016
(51) Int. Cl.: G01N 29/024, G01N 29/032, G01N 29/07, G01N 29/11, G01N 29/30, G01N 29/46, G01N 29/44

(54) **VORRICHTUNG ZUR UNTERSUCHUNG EINES KÖRPERGEWEBES DURCH AKUSTISCHE SPEKTROSKOPIE**
DEVICE FOR EXAMINING BODY TISSUE USING ACOUSTIC SPECTROSCOPY
DISPOSITIF DESTINÉ À L'ANALYSE D'UN TISSU CORPOREL PAR SPECTROSCOPIE ACOUSTIQUE

(30) Priorität: 17.07.2015 EP 15177198; 26.10.2015 DE 102015118226
(43) Veröffentlichungstag der Anmeldung: 30.05.2018
(73) Patentinhaber: Sonovum GmbH, 04103 Leipzig (DE)
(72) Erfinder: WROBEL, Miroslaw, 97753 Karlstadt (DE)
(74) Vertreter: advotec.
(86) Internationale Anmeldenummer: PCT/EP2016/063863
(87) Internationale Veröffentlichungsnummer: WO 2017/012792

(56) Entgegenhaltungen:
- DE-A1-102008 014 300
- DE-B3- 10 353 132
- DE-B3-102005 005 386
- DE-C1- 4 141 123
- DE-U1-202007 017 911
- US-A- 5 433 112
- US-A- 6 109 098
- US-A1- 2015 041 625
- JU BING-FENG ET AL: "Simultaneous measurement of local longitudinal and transverse wave velocities, attenuation, density, and thickness of films by using point-focus ultrasonic spectroscopy", JOURNAL OF APPLIED PHYSICS, AMERICAN INSTITUTE OF PHYSICS, US, Bd. 112, Nr. 8, 15. Oktober 2012 (2012-10-15), Seiten 84910-84910, XP012167758, ISSN: 0021-8979, DOI: 10.1063/1.4758136 [gefunden am 2012-10-25]
- HALL S J ET AL: "DIRECT TIME-TO-DIGITAL CONVERTER WITH MULTI-STOP FACILITY", NUCLEAR INSTRUMENTS AND METHODS,, vol. 140, 1 January 1977 (1977-01-01), pages 283-287, XP001436305,
- MAVIN SWAPP: "TIME TO DIGITAL CONVERTER RESOLVES PICOSECONDS IN TIME INTERVAL MEASUREMENTS", TECHNICAL DEVELOPMENTS,, vol. 2, 1 January 1982 (1982-01-01), page 53, XP001436857,
- GERDS E J ET AL: "A CMOS TIME TO DIGITAL CONVERTER IC WITH 2 LEVEL ANALOG CAM", IEEE JOURNAL OF SOLID-STATE CIRCUITS, IEEE, USA, vol. 29, no. 9, 1 September 1994 (1994-09-01), pages 1068-1076, XP000475950, ISSN: 0018-9200, DOI: 10.1109/4.309902
- KARI MAATTA ET AL: "A High-Precision Time-to-Digital Converter for Pulsed Time-of-Flight Laser Radar Applications", IEEE TRANSACTIONS ON INSTRUMENTATION AND MEASUREMENT, IEEE SERVICE CENTER, PISCATAWAY, NJ, US, vol. 47, no. 2, 1 April 1998 (1998-04-01), XP011024486, ISSN: 0018-9456

## Beschreibung

Die Erfindung betrifft eine Vorrichtung zur Untersuchung eines Körpergewebes durch akustische Spektroskopie

Ein Verfahren zur Bestimmung des Zustands eines Stoffgemisches mittels Messung der Schallgeschwindigkeit und/oder Ultraschalldämpfung offenbart die DE 10 2008 014 300 A1. Um aus Schallgeschwindigkeit und Dämpfung Rückschlüsse auf die Zusammensetzung des zu untersuchenden Stoffgemischs zu ziehen, werden dabei verfahrensgemäß die Amplituden der transmittierten und reflektierten Schallwellen ausgewertet und die Laufzeit der einzelnen Schallwellen aus der quadrierten Signalamplitude der gesendeten und empfangenen Signale berechnet.

Ju Bing-Feng et al. schlagen in ihrer Veröffentlichung mit dem Titel "Simultaneous measurement of longitudinal and transverse velocities, attenuation, density, and thickness of films by using point-focus ultrasonic spectroscopy" im Journal of Applied Physics ein weiteres Verfahren zur Bestimmung der akustischen und geometrischen Eigenschaften eines dünnschichtigen Prüfmaterials mittels Spektroskopie vor, bei dem die akustische Impedanz, der Dämpfungskoeffizient und die Durchlaufzeit der fokussierten Ultraschallwellen aus den Reflektionsspektren der Ultraschallwellen berechnet werden.

Zur kontinuierlichen Untersuchung eines (biologischen) Mediums ist aus der DE 20 2007 017 911 U1 eine Vorrichtung bekannt, die eine kegelförmige Abstrahlcharakteristik erzeugt, um anhand des Vergleichs eines Referenzmusters von Phasenverschiebungen mit den an einem Empfänger auftretenden Phasenverschiebungen eine Zustandsbeurteilung des Prüfmaterials zu ermöglichen; es ist auch möglich, zusätzlich zur Phasenverschiebung die Amplitudenänderung zwischen den Sende- und Empfangssignalkomponenten derselben Frequenz auszuwerten.

Aus der US 5 433 112 A sind ein weiteres Verfahren und eine Vorrichtung zur Charakterisierung einer Polymerschmelze bekannt, mit dem bzw. der die Zeitverschiebung zwischen zwei aufeinanderfolgenden aus einer Polymerschmelze austretenden Ultraschallechos ermittelt werden kann. Zur Berechnung der akustischen Materialkennwerte werden bei diesem Verfahren die auftretenden Amplitudenänderungen einbezogen.

Eine Vorrichtung zur Kombination der akustischen mit der elektroakustischen Spektroskopie ist der US 6 109 098 A zu entnehmen. Zur Charakterisierung dispergierter Systeme wird sowohl die Bestimmung der Teilchengrößenverteilung als auch des Zeta-Potentials des Systems vorgeschlagen.

Spektroskopische Vorrichtungen zeichnen eine Vielzahl an Daten auf, welche in der Folge zur Vereinfachung und/oder Beschleunigung der Weiterverarbeitung komprimiert und/oder reduziert werden. Verfahren zur Kompression und/oder Reduktion automatisiert erfasster Daten werden in den Druckschriften DE 103 53 132 B3, DE 41 41 123 C1 und DE 10 2005 005 386 B3 vorgeschlagen.

Eine weitere Vorrichtung zur Untersuchung von flüssigen oder gasförmigen Medien durch akustische Spektroskopie ist beispielsweise aus der DE 103 24 990 B2 bekannt. Bei dieser Vorrichtung sind eine Sendeeinrichtung zum Senden mehrerer Sendesignale unterschiedlicher Frequenz und eine Empfangseinrichtung zum Empfangen entsprechender Empfangssignale vorgesehen. Mit einer Verarbeitungseinrichtung kann dann die Phasenverschiebung zu jedem Sende- und Empfangssignalpaar ermittelt und daraus ein das untersuchte Medium qualifizierender Wert anhand der Phasenverschiebung abgeleitet werden. Bei der dort beschriebenen Vorrichtung werden nacheinander in unmittelbarer Abfolge Signalpakete unterschiedlicher Frequenzen gesendet. Die Frequenzen bewegen sich vorzugsweise im Bereich zwischen 1 und 15 MHz, wobei jedes Signalpaket vorzugsweise wenigstens 100 Perioden umfassen sollte. Zu jedem Signalpaket und damit zu jeder Frequenzstufe werden über die Empfangseinrichtung die spezifischen Empfangssignale aufgenommen und danach einer Verarbeitungseinrichtung zugeleitet. In der Verarbeitungseinrichtung wird zu jedem frequenzspezifischen Sende- und Empfangssignalpaar der durch den Durchtritt durch das untersuchte Medium verursachte Phasenverschiebungswinkel zwischen den beiden Signalen ermittelt. So wird im Endeffekt durch diese frequenzspezifische Abtastung des Mediums eine Vielzahl unterschiedlicher frequenzspezifischer Phasenverschiebungswerte erfasst, deren Anzahl abhängig von der Anzahl der Sende- und Empfangssignalpaare ist. Auf diese Weise erhält man Informationen über das Verhalten des Mediums aus einem sehr großen Frequenzbereich definiert über einzelne Frequenzstufen, wobei diese Informationen Aussagen über die Eigenschaften des untersuchten Mediums ermöglichen. Durch die frequenzspezifische Abtastung des Mediums und die entsprechende Bestimmung der frequenzbezogenen Phasenwinkel lässt sich nämlich eine wesentlich spezifischere Untersuchung des Mediums erreichen, da sich eine Änderung des Mediums unterschiedlich auf das jeweilige frequenzspezifische Sende- und Empfangssignalpaar auswirkt.

Nachteilig an der in der DE 103 24 990 B2 beschriebenen Vorrichtung ist es, dass sie auf der Auswertung der Phasenverschiebungswerte beruht, die durch den Durchtritt des Sendesignals durch das zu untersuchende Merkmal verursacht und durch das Empfangssignal dokumentiert wird. Denn die Auswertung der Phasenverschiebung zwischen Sendesignal und Empfangssignal kann zu unerwünschten Informationsverlusten führen, durch die das Auswertungsergebnis signifikant verfälscht wird. Diese Informationsverluste beruhen darauf, dass der Wert der Phasenverschiebung auf einen Wertebereich zwischen 0 und 360° begrenzt ist. Ist die Phasenverschiebung zwischen Sendesignal und Empfangssignal jedoch größer als 360°, so liefert das in der Druckschrift beschriebene Verfahren einen Phasenverschiebungswert, der nicht eindeutig zugeordnet werden kann. Beträgt die Phasenverschiebung zwischen Sendesignal und Empfangssignal beispielsweise 400°, so liefert die in der Druckschrift beschriebene Auswertevorrichtung einen Phasenverschiebungswert von 40°. Die daraus abgeleiteten Auswertungsergebnisse sind somit entscheidend verfälscht und unbrauchbar, da die Auswertung Ergebnisse liefert, die einer Phasenverschiebung von 40° entspricht und nicht einer Phasenverschiebung von 400° (= 360° + 40°).

Zur Lösung dieses Problems der nicht eindeutig zuordenbaren Ergebnisse bei der Auswertung der Phasenverschiebung zwischen Sendesignal und Empfangssignal schlägt die DE 198 41 154 A1 die Generierung von Sendesignalen mit unterschiedlicher Frequenz vor. Die verschiedenen Frequenzen dienen dabei in der Art eines Nonius bei der Bewertung der Phasenverschiebungen. So ist es möglich die gemessenen Phasenverschiebungswerte eindeutig der tatsächlichen Phasenverschiebung zwischen Sendesignal und Empfangssignal zuzuordnen. Dieses Verfahren hat jedoch den Nachteil, dass die Schallwellen in dem zu untersuchenden Medium abhängig von der jeweiligen Sendefrequenz mit unterschiedlicher Schallgeschwindigkeit übertragen werden, wobei diese verschiedenen Übertragungsgeschwindigkeiten im zu untersuchenden Medium zu erheblichen Messfehlern führen können. Die Abhängigkeit der Ausbreitungsgeschwindigkeit des Sendesignals von der jeweiligen Sendefrequenz wird durch die Kramers-Kronig-Gleichung beschrieben. Insofern hat sich das Messverfahren gemäß der DE 198 41 154 A1 als ungeeignet erwiesen, da die Messfehler aufgrund der nicht einheitlichen Ausbreitungsgeschwindigkeit der Schallwellen abhängig von der jeweiligen Sendefrequenz nicht berücksichtigt werden können.

Die Grundlagen der akustischen Spektroskopie, die der erfindungsgemäßen Vorrichtung zugrunde liegen, sind beispielsweise, jedoch keineswegs ausschließlich in folgenden Fachbüchern beschrieben:
Fachbuch 1: Molekularakustik, Werner Schaaffs, Springer Verlag, 1963 (ISBN-10:3642491413, ISBN-13:978-3642491412)
Fachbuch 2: Molecular Acustics/Molekularakustik, K.-H. Hellwege, A.M. Hellwege, W. Schaaffs, Springer Verlag, 1967 (ISBN-10:3540038973, ISBN-13:978-3540038979)
Fachbuch 3: Molecular Acustics, A.J. Matheson, John Willy & Sons Verlag, 1971 (ISBN-10:1861561857, ISBN-13:978-1861561855)

Ausgehend von dieser Vorrichtung soll eine Vorrichtung zur nichtinvasiven Untersuchung von Materialen durch akustische Spektroskopie vorgeschlagen werden. Erfindungsgemäß ist das zu untersuchende Material Körpergewebe. Bei der Untersuchung von Körpergewebe, insbesondere, jedoch keineswegs ausschließlich, zu diagnostischen Zwecken, besteht vielfach das Problem, dass das Körpergewebe für die Untersuchung nicht ohne Weiteres entnommen werden kann, so dass nur nichtinvasive Untersuchungsmethoden zum Einsatz kommen können. Sollen beispielsweise Untersuchungen am menschlichen Gehirn vorgenommen werden, so ist eine Entnahme von Körpergewebe aufgrund der unübersehbaren Nebenwirkungen bei der Öffnung des menschlichen Schädels in den allermeisten Fällen nicht möglich.

Aufgabe der vorliegenden Erfindung ist es, eine Vorrichtung zur nichtinvasiven Untersuchung von Körpergewebe durch akustische Spektroskopie vorzuschlagen, bei der die oben beschriebenen Fehlerquellen ausgeschlossen werden.

Diese Aufgabe wird durch eine Vorrichtung nach der Lehre des Anspruchs 1 gelöst.

Vorteilhafte Ausführungsformen der Erfindung sind Gegenstand der Unteransprüche.

Die erfindungsgemäße Vorrichtung zur nichtinvasiven Untersuchung eines Körpergewebes durch akustische Spektroskopie umfasst eine Ultraschall-Sendeeinrichtung zum Aussenden von Ultraschall-Sendesignalen und eine Ultraschall-Empfangseinrichtung zum Empfang der reflektierten bzw. transmittierten Ultraschall-Empfangssignale nach Durchlaufen des zu untersuchenden Körpergewebes. Sowohl die Sendeeinrichtung als auch die Empfangseinrichtung sind dabei zum Senden bzw. Empfangen von Ultraschallsignalen unterschiedlicher Frequenzen fi bis fₙ geeignet. Erfindungsgemäß umfasst die Vorrichtung eine erste Verarbeitungseinrichtung, eine zweite Verarbeitungseinrichtung, eine zwischen der Ultraschall-Empfangseinrichtung einerseits und den beiden Verarbeitungseinrichtungen andererseits vorgesehene Signalweiche, eine Speichereinrichtung und eine Auswerteeinrichtung. Mit der ersten einen Time-to-digital-Converter umfassenden Verarbeitungseinrichtung sind die frequenzabhängigen Durchlaufzeitwerte des zugeordneten Ultraschallsignals eines jeden Sende- und Empfangssignalpaares unmittelbar messbar, mit der zweiten Verarbeitungseinrichtung sind aus jedem Sende- und Empfangssignalpaar die frequenzabhängigen Dämpfungswerte des zugeordneten Ultraschallsignals ermittelbar. Mit der Signalweiche ist ein jeweils empfangenes Ultraschall-Empfangssignal identisch zur parallelen Verarbeitung auf beide Verarbeitungseinrichtungen leitbar. In der Speichereinrichtung sind zu jeder Frequenz fi bis fₙ der verschiedenen Sende- und Empfangssignale, wobei n größer 1 ist, die jeweils ermittelten Durchlaufzeitwerte und Dämpfungswerte zumindest zeitweise als Ergebnisdatensatz abspeicherbar, in der Auswerteeinrichtung ist aus dem Ergebnisdatensatz ein qualifizierender Wert zur Beschreibung des untersuchten Körpergewebes ableitbar.

In Abkehr der Lehre aus der DE 103 24 990 B3 wird bei der Signalauswertung jedoch nicht die Phasenverschiebung zu jedem Sende- und Empfangssignalpaar bestimmt. Stattdessen wird erfindungsgemäß in der ersten Verarbeitungseinrichtung aus jedem Sende- und Empfangssignalpaar die Durchlaufzeit des zugeordneten Ultraschallsignals ermittelt. Daneben wird in der zweiten Verarbeitungseinrichtung aus jedem Sende- und Empfangssignalpaar die Dämpfung des zugeordneten Ultraschallsignals beim Durchlauf durch das zu untersuchende Körpergewebe ermittelt. Dies bedeutet also mit anderen Worten, dass mit der erfindungsgemäßen Vorrichtung aus jedem Sende- und Empfangssignalpaar einer bestimmten Frequenz als Ergebnis ein Datenpaar aus der jeweiligen Durchlaufzeit und der jeweiligen Dämpfung ermittelt und in der Speichereinrichtung zu der jeweiligen Frequenz abgespeichert wird. Es ist dabei erfindungsgemäß nicht notwendig alle Daten zur Beschreibung eines Paares aus Sendesignal und Empfangssignal zu erfassen. Beispielsweise ist es nicht notwendig die Phase des Sendesignals zu kennen, da die Phase des Sendesignals für die Ermittlung von Durchlaufzeit und Dämpfung des Sendesignals beim Durchlauf durch das zu untersuchende Körpergewebe nicht erforderlich ist. Es müssen also nur die Daten zur Beschreibung von Sendesignal und Empfangssignal erfasst werden, die für die Ermittlung von Durchlaufzeit und Dämpfung notwendig sind.

Denn Untersuchungen an Versuchsmaterialen, beispielsweise an menschlichen Körpergewebe, bei der Beaufschlagung mit Ultraschallsignalen und der Ermittlung der Durchlaufzeit zum einen und der Dämpfung zum anderen für jedes einzelne Sende- und Empfangssignalpaar haben gezeigt, dass Veränderungen im Material, d.h. im Körpergewebe, sich signifikant in den Ergebnisdatenpaaren aus Durchlaufzeit und Dämpfung abbilden. Die konventionelle akustische Spektroskopie, bei der die Signaldämpfung zu messen ist, wird also erfindungsgemäß dahingehend erweitert, dass nicht nur für jede Frequenz die spezifische und frequenzabhängige Dämpfung (Attenuation ATN), sondern auch die spezifische und frequenzabhängige Durchlaufzeit (Time of Flight ToF) bestimmt wird. Durch die Messung der Durchlaufzeit kann nämlich die Dispersivität des untersuchten Materials ermittelt werden. Die Kombination der Messung von Dämpfung und Dispersivität des untersuchten Materials lässt eine sehr differenzierte Charakterisierung des untersuchten Materials zu.

Untersuchungen einer Vielzahl von Untersuchungspersonen haben beispielsweise gezeigt, dass Gewebeänderungen im menschlichen Gehirn mit Änderungen des dispersiven Charakters des Gehirngewebes einhergehen, die mit der erfindungsgemäßen Vorrichtung ermittelt werden können. So kann das Schlaganfallrisiko eines Patienten als Folge von Gewebeänderungen im Gehirn erfasst und quantitativ abgeschätzt werden. Auch bei der Alzheimer-Erkrankung oder der Parkinson-Erkrankung kommt es zu Gewebeänderungen im Gehirn, die mit signifikanten Änderungen bei der Durchlaufzeit und der Dämpfung bei der Beaufschlagung mit Ultraschallsignalen einhergehen und mit der erfindungsgemäßen Vorrichtung ermittelt werden können. Somit stellt die erfindungsgemäße Vorrichtung ein Gerät zur Verfügung, mit dem pathologische Änderungen im Körpergewebe mittels nichtinvasiver, akustischer Spektroskopie ermittelt und quantitativ abgeschätzt werden können.

Bei der Nutzung der erfindungsgemäßen Vorrichtung zur nichtinvasiven Untersuchung von Materialien fallen sehr große Ergebnisdatensätze an, da für jede Frequenz und das zugeordnete Sende- und Empfangssignalpaar ein Ergebnisdatensatz mit der jeweiligen Durchlaufzeit und der jeweiligen Dämpfung bestimmt und abgespeichert wird. Werden sehr viele Untersuchungen durchgeführt und soll eine Auswertung dieser Daten durch Vergleich der Ergebnisdaten mit Daten aus einer zuvor aufgebauten Ergebnisdatenbank ermittelt werden, kann es aufgrund der großen Datenmengen leicht zu Verarbeitungsproblemen kommen. Es ist deshalb besonders vorteilhaft, wenn in einer Datenreduktionseinrichtung aus dem ermittelten Ergebnisdatensatz ein reduzierter Ergebnisdatensatz abgeleitet werden kann, wobei der reduzierte Ergebnisdatensatz den ermittelten Ergebnisdatensatz charakteristisch abbildet und einen geringeren Datenumfang hat. Durch diese Datenreduktion der Ergebnisdaten kann die Datenverarbeitung bei der Auswertung der Ergebnisdaten erheblich beschleunigt werden.

In welcher Weise die Datenreduktionseinrichtung die Ergebnisdatensätze reduziert ist grundsätzlich beliebig. Gemäß einer ersten Variante wird dazu in einem Ergebnisdatensatz, der alle Sendefrequenzen, und alle Sende- und Empfangssignalpaare für die Untersuchung an einem Material enthält, die jeweils maximale und minimale Durchlaufzeit sowie die jeweils maximale und minimale Dämpfung bestimmt. Diese zwei Maximal- und zwei Minimalwerte bilden dann einen reduzierten Ergebnisdatensatz. Diese Datenreduktion kann als charakteristisch für den ursprünglichen Datensatz angenommen werden, da der von den beiden Maximalwerten und den beiden Minimalwerten aufgespannte rechteckige Datenquadrant alle anderen Ergebnisdaten umfasst.

Eine noch stärkere Datenreduktion kann erreicht werden, wenn in der Datenreduktionseinrichtung nicht nur die Ergebnisdaten eines Ergebnisdatensatzes, sondern die Ergebnisdaten mehrerer Ergebnisdatensätze zusammengefasst werden. Wurden beispielweise Untersuchungen an einer Vielzahl von Personen durchgeführt, so können beispielsweise die Ergebnisdaten aus den Ergebnisdatensätzen aller männlichen Untersuchungspersonen und zum anderen die Ergebnisdaten aus den Ergebnisdatensätzen aller weiblichen Untersuchungspersonen zusammengefasst werden. Die reduzierten Ergebnisdatensätze repräsentieren dann die Ergebnisdaten zum einen der männlichen Untersuchungspersonen und zum anderen der weiblichen Untersuchungspersonen. In gleicher Weise können beispielsweise auch die Ergebnisdatensätze unterschiedlicher Altersklassen zusammengefasst werden. Welche Klassen bei der Ergebnisdatenreduktion dabei jeweils zusammengefasst werden, ist grundsätzlich beliebig und lediglich abhängig vom jeweils gewünschten Untersuchungsergebnis. Um die Ergebnisdaten aus mehreren ermittelten Ergebnisdaten zusammenzufassen, kann mit der Datenreduktionseinrichtung jeweils der maximale und der minimale Durchlaufzeitwert und der maximale und der minimale Dämpfungswert in all den eine Klasse repräsentierten Ergebnisdatensätzen bestimmt werden. Die beiden Maximalwerte bzw. die beiden Minimalwerte spannen dann einen Ergebnisdatenraum auf, der die Ergebnisdaten aus allen zusammengefassten Ergebnisdatensätzen umfasst und repräsentiert.

Eine weitere Möglichkeit zur Datenreduktion besteht darin, in der Datenreduktioneinrichtung zumindest ein Quantil der Durchlaufzeitwerte in einem ermittelten Ergebnisdatensatz sowie zumindest ein Quantil der Dämpfungswerte in diesem Ergebnisdatensatz zu bestimmen. Diese Quantile werden dann als reduzierter Ergebnisdatensatz gespeichert. Als Quantil soll im Sinne der Erfindung ein statistischer Schwellwert in der Menge der Durchlaufzeitwerte bzw. der Menge der Dämpfungswerte eines oder mehrerer Ergebnisdatensätze verstanden werden.

In welcher Weise die mittels der erfindungsgemäßen Vorrichtung ermittelten Ergebnisdatensätze bzw. die daraus abgeleiteten reduzierten Ergebnisdatensätze verwendet werden, ist grundsätzlich beliebig. Besonders vorteilhaft ist es, wenn mittels der Vorrichtung eine Auswertung vorgenommen werden kann, die einen das Material, beispielsweise ein Körpergewebe, qualifizierenden Wert ergibt. Durch die frequenzspezifische Abtastung des Mediums und die entsprechende Bestimmung der Dämpfung zum einen und der Durchlaufzeit zum anderen lässt sich eine spezifische Untersuchung und die Ableitung von das untersuchte Material qualifizierenden Werten problemlos erreichen, da sich eine Änderung des Mediums in ganz signifikanter Weise auf die jeweiligen Ergebnisdatensätze auswirkt.

Für die Beurteilung, Klassifizierung und Modulierung bei der Auswertung der Ergebnisdatensätze bzw. bei der Auswertung der reduzierten Ergebnisdatensätze gibt es beispielsweise folgende Methoden:
- Multiple Linear Regression (MLR)
- Principle Component Regression (PCR)
- Partial Least Square Regression (PLSR)
- L-shaped Partial Least Square Regression (L-PLSR)
- Support Vector Machine Regression (SVM-R)

In welcher Weise in der Auswerteeinrichtung aus den Ergebnisdatensätzen bzw. aus den reduzierten Ergebnisdatensätzen der qualifizierende Wert zur Beschreibung des untersuchten Materials abgeleitet wird, ist grundsätzlich beliebig. Besonders vorteilhaft ist es, wenn dies in der Weise eines Mustervergleichs erfolgt, bei dem die Ergebnisdatensätze mit Vergleichs-Ergebnisdatensätzen aus einer Datenbank verglichen werden. Die Vergleichs-Ergebnisdatensätze repräsentieren dabei Messungen an Vergleichsmaterialen mit bekannten Eigenschaften. Dabei kann es sich um Körpergewebe von Patienten mit bekannten Erkrankungen, beispielsweise Alzheimer, Parkinson oder Bluthochdruck handeln. Auch ist es denkbar, dass die Vergleichs-Ergebnisdatensätze repräsentierend bei Messungen an Zellgewebe mit speziellen Markierungen in den Zellen gemacht wurden.

Diese Art des Mustervergleichs führt zu sehr differenzierten Untersuchungsergebnissen, da die mit der erfindungsgemäßen Vorrichtung ermittelten Ergebnisdatensätze für jedes Material abhängig von seiner spezifischen Eigenschaft eine Art Fingerabdruck liefern, wobei die Ergebnisdatenpaare aus Durchlaufzeit und Dämpfung für die verschiedenen Frequenzen ein Datenmuster bilden, das das untersuchte Material und dessen Eigenschaften charakteristisch beschreiben kann. Mit jeder Änderung im untersuchten Material geht eine Änderung dieses vom Ergebnisdatensatz repräsentierten Fingerabdrucks einher. Wird der vom Ergebnisdatensatz repräsentierte Fingerabdruck mit den Fingerabdrücken der Vergleichs-Ergebnisdatensätze in der Datenbank verglichen, kann durch diese Mustervergleichsmethode eine sehr differenzierte Aussage über das jeweils untersuchte Materials getroffen werden. Derartige Mustervergleiche bedürfen sehr häufig der Klassifikation der Ergebnisdatensätze, wozu folgende Methoden angewendet werden können:
- Principle Component Analysis (PCA)
- Partial Least Square Analysis (PLSA)
- Linear Discriminante Analysis (LDA)
- Support Vector Machine Classification (SVM-C)
- Partial Least Square Discriminante Analysis (PLS-DA)
- Soft Independent Modelling of Class Analogy (SIMCA)

Mit der erfindungsgemäßen Vorrichtung wird in Abkehr der bisherigen Untersuchungsmethoden nicht mehr die Phasenverschiebung, sondern die Durchlaufzeit des Ultraschallsignals selber bestimmt. Um die Eigenschaften des untersuchten Materials mit einer ausreichenden Auflösung bestimmen zu können, ist es besonders vorteilhaft, wenn die Durchlaufzeit mit einer Auflösung von zumindest 100 Pikosekunden in der ersten Verarbeitungseinrichtung ermittelt werden kann. Bevorzugt sollte die Durchlaufzeit mit einer Auflösung von zumindest 10 Pikosekunden ermittelt werden können.

Welche Zeitmesseinrichtung in der ersten Verarbeitungseinrichtung zur Bestimmung der Durchlaufzeit vorgesehen ist, ist grundsätzlich beliebig. Um Auflösungen bei der Messung der Durchlaufzeit, insbesondere im Bereich von unter 10 Pikosekunden, zu erreichen, ist es erfindungswesentlich, wenn ein Time-to-digital-Converter eingesetzt wird. Bei diesen Time-to-digital-Convertern handelt es sich um elektronische Baugruppen, die Zeitintervalle im Bereich von kleiner 100 Pikosekunden messen und in eine digitale Ausgabe umwandeln können. Die Messung der Zeit beruht dabei auf der bekannten Durchlaufzeit eines elektrischen Signals durch die Baugruppen des TDC-Converters. Die Durchlaufzeit wird dabei dann dadurch bestimmt, wie viele der elektronischen Schaltkreise mit bekannter Durchlaufzeit zwischen der Abgabe des Sendesignals zum einen und dem Empfang des Eingangssignals zum anderen durchlaufen werden. Diese Anzahl der Durchläufe von elektronischen Baugruppen mit bekannter Durchlaufzeit wird dann gezählt, wobei die gemessene Durchlaufzeit dann der Multiplikation der bekannten Durchlaufzeit eines einzelnen Schaltkreises mit der Anzahl von Durchläufen entspricht.

Für eine korrekte Auswertbarkeit der Ergebnisdatensätze ist es von großer Bedeutung, dass der Durchlaufzeitwert und der Dämpfungswert anhand des identischen Sende- und Empfangssignalpaars ermittelt werden. Denn bereits kleinste Änderungen in der Eigenschaft des untersuchten Körpergewebes, beispielsweise aufgrund des sich verändernden Blutdrucks in einem Körpergewebe, können dazu führen, dass die Ergebnisdaten verfälscht werden, wenn Dämpfung und Durchlaufzeit hintereinander und nicht anhand des identischen Sende- und Empfangssignalpaars bestimmt werden. Um dies zu ermöglichen enthält die erfindungsgemäße Vorrichtung eine Signalweiche, mit der ein jeweils empfangenes Ultraschall-Empfangssignal identisch zur parallelen Verarbeitung auf die erste Verarbeitungseinrichtung zur Bestimmung der Durchlaufzeit und die zweite Verarbeitungseinrichtung zur Bestimmung der Dämpfung geleitet wird.

Erfindungsgemäß geeignet ist die Vorrichtung zur nichtinvasiven Untersuchung von Körpergewebe. In diesem Fall wird das Körpergewebe eines zu untersuchenden Körperteils mit den Ultraschall-Sendesignalen beaufschlagt und die Ultraschall-Empfangssignale nach Durchlaufen des Körpergewebes aufgezeichnet. Besonders große Vorteile bietet die erfindungsgemäße Vorrichtung bei der Untersuchung am menschlichen Gehirn, da Untersuchungen am menschlichen Gehirn, insbesondere molekulare oder zellulare Untersuchungen, ansonsten bisher gar nicht oder nur mit sehr geringer Aussagekraft durchgeführt werden können. Da eine Öffnung des menschlichen Schädels zur Untersuchung des Gehirngewebes aufgrund der erheblichen Nebenwirkungen in den aller meisten Fällen nicht möglich ist, können Untersuchungen des Gehirngewebes bisher in der Regel nur mittels bildgebender Verfahren, wie der Magnetresonanztherapie oder der Computertomographie, durchgeführt werden. Diese bildgebenden Verfahren erlauben aber regelmäßig keine differenzierten Diagnoseaussagen über die meisten Krankheitsbilder im Gehirn. Insbesondere können beispielsweise Alzheimer-Erkrankungen oder Parkinson-Erkrankungen mittels der bildgebenden Untersuchungsverfahren erst in einem Stadium festgestellt werden, in dem eine wirksame Therapie in den meisten Fällen nicht mehr möglich ist. Um die Untersuchung des Gehirngewebes mit der erfindungsgemäßen Vorrichtung vornehmen zu können, sollte die Ultraschall-Sendeeinrichtung bzw. die Ultraschall-Empfangseinrichtung zur temporären und/oder dauerhaften Anordnung an der Außenseite der Kopfhaut des Patienten geeignet sein. Für diese Anbringung der Ultraschall-Sendeeinrichtung bzw. der Ultraschall-Empfangseinrichtung an der Kopfhaut bedarf es dann regelmäßig keines Arztes, sondern die Sende- und Empfangseinrichtung kann auch durch entsprechend ausgebildetes Pflegepersonal oder sogar durch Laien erfolgen.

Welche Art von Ultraschallwellen zur Untersuchung mittels der erfindungsgemäßen Vorrichtung eingesetzt werden, ist grundsätzlich beliebig. Besonders geeignet sind Longitudinalultraschallwellen im unteren MHz-Bereich.

Weiterhin ist es denkbar, dass die Ultraschall-Sendesignale unterschiedlicher Frequenz von der Ultraschall-Sendeeinrichtung einzeln nacheinander oder als Gruppen nacheinander ausgesendet werden.

Alternativ dazu kann das Ultraschall-Sendesignal für die unterschiedlichen Frequenzen auch mittels der Ultraschall-Sendeeinrichtung auf ein gemeinsames Trägersignal aufmodelliert und ausgesendet werden. Auch ist es möglich, dass die Ultraschall-Sendesignale unterschiedlicher Frequenz in der Ultraschall-Sendeeinrichtung auf ein gemeinsames Trägersignal als Superposition aufaddiert und gemeinsam ausgesendet werden.

Erfindungsgemäß werden von der Sendeeinrichtung Sendesignale mit unterschiedlichen Frequenzen erzeugt, um für jede dieser Frequenzen ein Sende- und Empfangssignalpaar zu ermitteln und zur Charakterisierung des untersuchten Materials auszuwerten. Neben der Änderung der Frequenz kann es zusätzlich nützlich sein mit der Sendeeinrichtung auch Ultraschall-Sendesignale mit jeweils unterschiedlichen Amplituden auszusenden. Damit lassen sich die Daten noch differenzierter auswerten.

Nachfolgend soll die Erfindung anhand einer in den Zeichnungen schematisch dargestellten Ausführungsform beispielhaft erläutert werden.

Es zeigt:
- **Fig. 1**: eine erfindungsgemäße Vorrichtung mit ihren verschiedenen Funktionsmodulen als Prinzipskizze;
- **Fig. 2**: eine Prinzipskizze zur Erläuterung der Arbeitsweise einer ersten Datenreduktionseinrichtung;
- **Fig. 3**: eine Prinzipskizze zur Erläuterung des Aufbaus einer Datenbank mit Vergleichs-Ergebnisdatensätzen;
- **Fig. 4**: eine Prinzipskizze zur Erläuterung der Arbeitsweise einer Auswerteeinrichtung der Vorrichtung gemäß Fig. 1 unter Verwendung einer Datenbank gemäß Fig. 3;
- **Fig. 5**: den Signalverlauf und die Signalfrequenzen eines Ultraschall-Sendesignals;
- **Fig. 6**: den Signalverlauf und die Signalfrequenzen eines zweiten Ultraschall-Sendesignals;
- **Fig. 7**: den Signalverlauf und die Signalfrequenzen eines dritten Ultraschall-Sendesignals;
- **Fig. 8**: den Signalverlauf und die Signalfrequenzen eines vierten Ultraschall-Sendesignals;
- **Fig. 9**: den Signalverlauf und die Signalfrequenzen eines fünften Ultraschall-Sendesignals;
- **Fig. 10**: den Signalverlauf und die Signalfrequenzen eines sechsten Ultraschall-Sendesignals;
- **Fig. 11**: den Signalverlauf und die Signalfrequenzen eines siebten Ultraschall-Sendesignals.

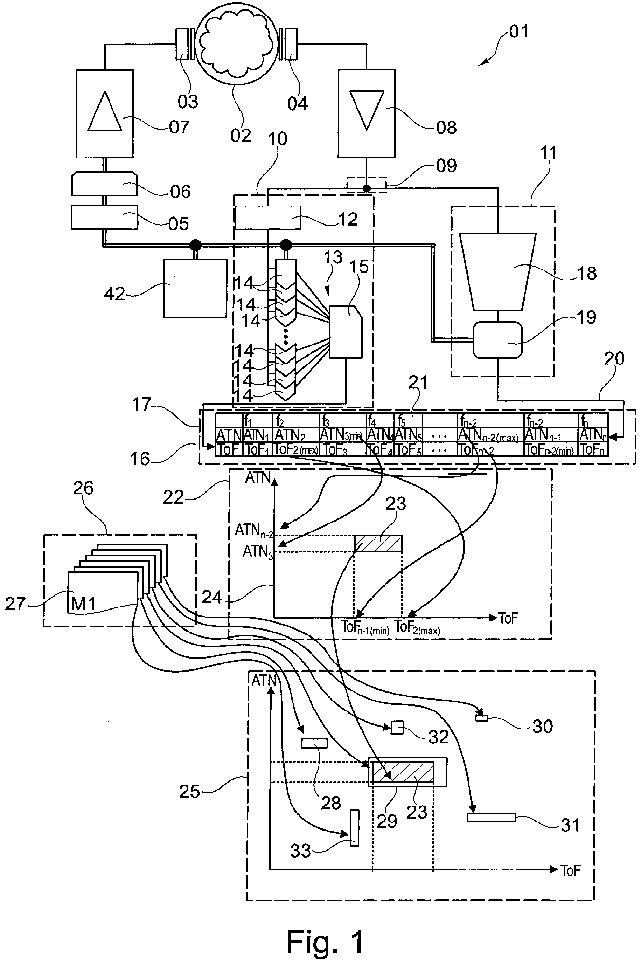

**Fig. 1** zeigt in einer Prinzipskizze den prinzipiellen Aufbau einer erfindungsgemäßen Vorrichtung 01, wie sie zur nichtinvasiven Untersuchung eines Materials 02, beispielsweise einem Gehirn, beispielsweise einem Schädel, durch Methoden der akustischen Spektroskopie eingesetzt werden kann. Die Vorrichtung 01 umfasst zum einen eine Ultraschall-Sendeeinrichtung 03 und eine Ultraschall-Empfangseinrichtung 04. Weiter umfasst die Vorrichtung 01 zur Generierung der an der Ultraschall-Sendeeinrichtung 03 abzustrahlenden Ultraschallsignale ein Signalvorbereitungsmodul 05, einen Signalgenerator 06 und einen Signalverstärker 07.

Nach direktem Durchlaufen oder indirektem Durchlaufen (Reflexion) des Körpergewebes 02 werden die Ultraschallsignale an der Ultraschallempfangseinrichtung 04 aufgefangen und mittels eines Signalverstärkers 08 verstärkt. Die verstärkten Empfangssignale werden anschließend in einer Signalweiche 09 aufgeteilt und zur parallelen Verarbeitung auf eine erste Verarbeitungseinrichtung 10 und eine zweite Verarbeitungseinrichtung 11 verteilt. Die erste Verarbeitungseinrichtung 10 dient der Bestimmung der Durchlaufzeiten, die das Ultraschallsignal bei der jeweils eingestellten Frequenz zum Durchlauf durch das Körpergewebe 02 benötigt. Nach Durchlauf eines Signalaufbereitungsmoduls 12 gelangt das Empfangssignal in einen Time-to-digital-Converter 13, mit dem die Durchlaufzeit des Ultraschallsignals, d.h. die Zeit zwischen der Abstrahlung an der Ultraschall-Sendeeinrichtung 03 bis zum Empfang des Ultraschallsignals an der Ultraschall-Empfangseinrichtung 04, gemessen werden kann. Die Funktion des Time-to-digital-Converters 13 beruht dabei darauf, dass die Durchlaufzeit eines elektrischen Signals durch eine Vielzahl von in dem Time-to-digital-Converter 13 enthaltenen elektronischen Schaltkreisen 14 bekannt ist.

Zur Zeitmessung der Durchlaufzeit wird das Auslösesignal des Ultraschall-Sendesignals zeitgleich mit der Abstrahlung an der Ultraschall-Sendeeinrichtung 03 auch auf den Time-to-digital-Converter 13 geleitet, um den Zeitmessprozess zu starten. Das Auslösesignal des Ultraschall-Sendesignals durchläuft dann die hintereinander angeordneten Schaltkreise 14 im Time-to-digital-Converter 13. Jeder Durchlauf eines elektronischen Schaltkreises 14, der einer vorbestimmten Durchlaufzeit entspricht, wird dabei von einem Zähler 15 aufaddiert. Sobald dann das Empfangssignal auf die elektronischen Schaltkreise 14 geleitet wird, schaltet der Zähler 15 ab und multipliziert die Anzahl der aufaddierten Durchläufe mit der bekannten Durchlaufzeit der einzelnen elektronischen Schaltkreise 14. Daraus ergibt sich dann insgesamt die Durchlaufzeit 16 für ein Sende- und Empfangssignalpaar. Für jede Sendefrequenz ergibt sich eine Durchlaufzeit 16, die zugehörig zu der jeweiligen Sendefrequenz in einer Speichereinrichtung 17 abgespeichert wird.

Parallel dazu wird in der zweiten Verarbeitungseinrichtung 11 die Signaldämpfung des Ultraschall-Sendesignals bei Durchlauf durch das Körpergewebe 02 bestimmt. Dazu ist in der zweiten Verarbeitungseinrichtung 11 ein Signalaufbereitungsmodul 18 vorgesehen, mit dem das Signal aufbereitet und die Amplitude des Ultraschall-Empfangssignals ermittelt wird. Anschließend wird in einem Auswertemodul 19 die Amplitude des Ultraschall-Empfangssignals mit der Amplitude des Ultraschall-Sendesignals verglichen. Durch diesen Vergleich kann in der zweiten Verarbeitungseinrichtung 11 zu jeder Frequenz eines Sende- und Empfangssignalpaars die zugehörige Dämpfung 20 bestimmt und in der Speichereinrichtung 17 abgespeichert werden.

Bei Durchführung einer Untersuchung am Körpergewebe 02 werden von einer Steuerung 42 vordefinierte Signalverläufe mit verschiedenen Sendefrequenzen definiert und anschließend von der Ultraschall-Sendeeinrichtung 03 abgestrahlt. Für jede einzelne Frequenz wird durch Auswertung des daraus generierten Sende- und Empfangssignalpaars die Durchlaufzeit 16 und der Dämpfungswert 20 bestimmt und in der Speichereinrichtung 17 abgespeichert. Am Ende des Messprozesses am Körpergewebe 02 ist in der Speichereinrichtung 17 ein Ergebnisdatensatz 21 abgespeichert. Der Ergebnisdatensatz 21 enthält für jede der Frequenzen fₗ bis fₙ die zugehörige Durchlaufzeit 16 und den zugehörigen Dämpfungswert 20, so dass der Ergebnisdatensatz 21 einen durch akustische Spektroskopie ermittelten Fingerabdruck der Materialeigenschaften des Körpergewebes 02 repräsentiert.

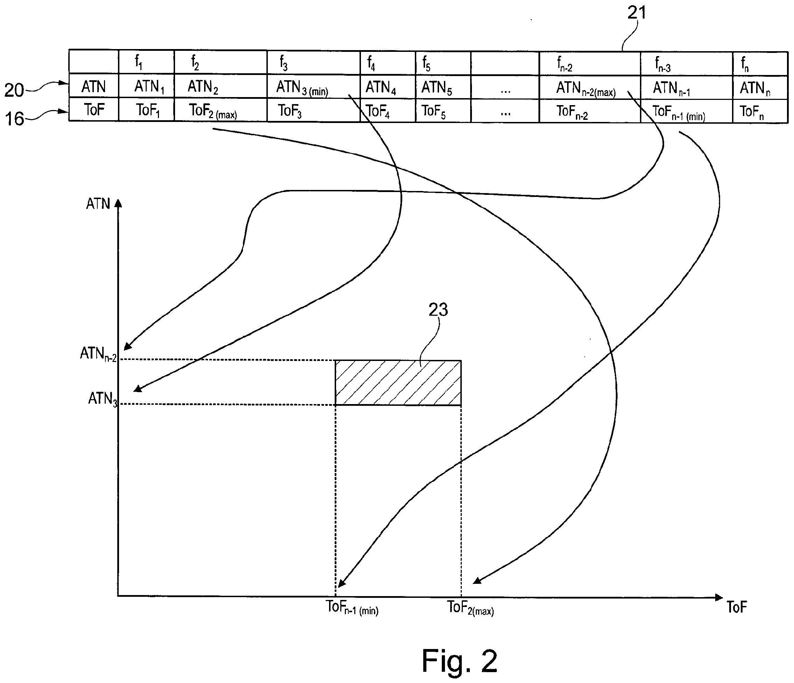

Der Speichereinrichtung 17 ist eine Datenreduktionseinrichtung 22 nachgeordnet, deren Funktionsweise nachfolgend anhand der Prinzipskizze in Fig. 2 erläutert werden soll.

**Fig. 2** stellt beispielhaft einen Ergebnisdatensatz 21 dar, in dem zu jeder Sendefrequenz fi bis fₙ die zugehörigen Durchlaufzeiten (ToF) und die zugehörigen Dämpfungswerte (ATN) gespeichert werden. Der Ergebnisdatensatz 21 kann dabei durchaus eine sehr hohe Anzahl von Daten enthalten, wenn beispielsweise an einem Körpergewebe 02 für mehrere hundert Frequenzen die jeweilige Durchlaufzeit und Dämpfung bestimmt würde. Um die Auswertung des Ergebnisdatensatzes 21 zu erleichtern, kann in der Datenreduktionseinrichtung 22 eine Datenreduktion wie folgt durchgeführt werden. Aus den im Ergebnisdatensatz 21 gespeicherten Durchlaufzeitwerten 16 und den gespeicherten Dämpfungswerten 20 werden jeweils die Maximal- und Minimalwerte bestimmt. Im in Fig. 2 dargestellten Beispiel stellt der Dämpfungswert ATN₃ das Dämpfungswertminimum und der Dämpfungswert ATNₙ₋₂ das Dämpfungswertmaximum dar. Weiter stellt der Durchlaufzeitwert ToF₂ das Durchlaufzeitmaximum und der Durchlaufzeitwert ToFₙ₋ₗ das Durchlaufzeitminimum dar.

Diese zwei Minimalwerte und die zwei Maximalwerte jeweils für Durchlaufzeit und Dämpfung stellen einen reduzierten Ergebnisdatensatz 23 dar, der bei Antragung in einem Koordinatensystem 24 durch ein Rechteck repräsentiert wird. Da der reduzierte Ergebnisdatensatz 23 durch die beiden Minimalwerte und die beiden Maximalwerte aus den verschiedenen Dämpfung- und Durchlaufzeitwerten abgeleitet wurde, lässt sich sagen, dass das den reduzierten Ergebnisdatensatz repräsentierende Rechteck 23 mit seiner Innenfläche alle anderen Daten des Ergebnisdatensatzes 21 umfasst und damit repräsentiert.

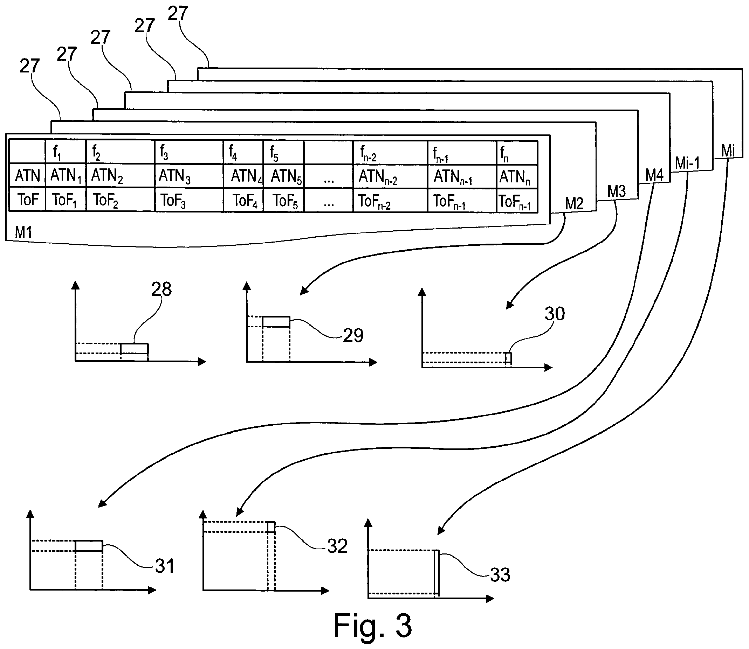

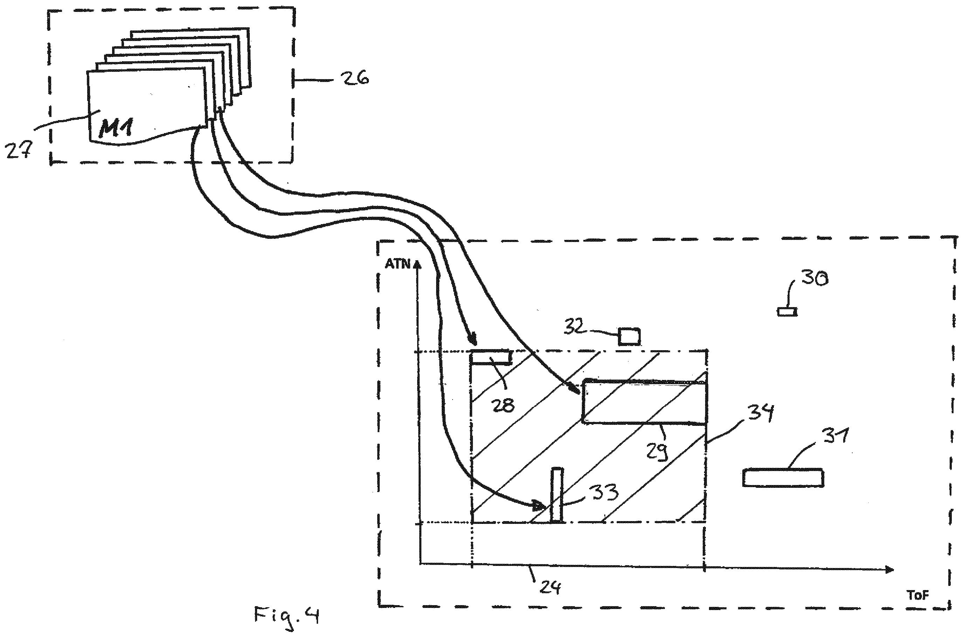

Wie aus **Fig. 1** ersichtlich, ist der Datenreduktionseinrichtung 22 eine Auswerteeinrichtung 25 nachgeordnet, die mit einer Datenbank 26 bei der Auswertung der reduzierten Ergebnisdatensätze 23 zusammenwirkt. In der Datenbank 26 ist dabei eine Vielzahl von Vergleichsergebnisdatensätzen 27 gespeichert, die durch Messung an Vergleichskörperteilen mit bekannten Eigenschaften bestimmt wurden. Die Arbeitsweise der Auswerteeinrichtung 25 in Zusammenwirkung mit der Datenbank 26 soll nachfolgend anhand der Prinzipskizzen in Fig. 3 und Fig. 4 näher erläutert werden.

**Fig. 3** zeigt zunächst die Datenbank 26 mit den verschiedenen Vergleichsergebnisdatensätzen 27. Jeder Vergleichsergebnisdatensatz 27 wurde durch eine akustische Spektroskopieuntersuchung an einem Vergleichskörperteil mit bekannten Eigenschaften ermittelt.

Um den Mustervergleich des reduzierten Ergebnisdatensatzes 21, der bei Messungen am Körpergewebe 02 bestimmt wurde, mit den Daten der Vergleichsdatensätze 27 in der Datenbank 26 zu ermöglichen, wird zunächst für jeden der Vergleichsergebnisdatensätze eine Datenreduktion durchgeführt, wie sie oben in Fig. 2 erläutert wird. Dies bedeutet mit anderen Worten, dass für jeden Vergleichsergebnisdatensatz ein reduzierter Ergebnisdatensatz 28, 29, 30, 31, 32 und 33 bestimmt wird, der jeweils im Koordinatensystem aus Durchlaufzeit und Dämpfung durch ein Rechteck repräsentiert wird.

Wie aus Fig. 1 ersichtlich, wird in der Auswerteeinrichtung 25 dann ein Mustervergleich des reduzierten Ergebnisdatensatzes 23, der das Körpergewebe 02 repräsentiert, mit den reduzierten Vergleichsergebnisdatensätzen 28 bis 30 durchgeführt. Wie in Fig. 1 beispielhaft dargestellt, zeigt der reduzierte Ergebnisdatensatz 23 des Körpergewebes 02 die größten Übereinstimmungen mit dem reduzierten Vergleichsergebnisdatensatz 29. Dadurch kann in der Auswerteeinrichtung 25 das Körpergewebe 02 als in seinen Eigenschaften vergleichbar mit den Eigenschaften des Vergleichskörperteils, das durch den reduzierten Ergebnisdatensatz 29 repräsentiert wird, qualifiziert werden. Dies bedeutet also mit anderen Worten, dass an der Auswerteeinrichtung 25 nach Durchführung des Mustervergleichs angezeigt werden kann, dass das Körpergewebe 02 signifikant mit dem vom reduzierten Vergleichsergebnisdatensatz 29 repräsentierten Körpergewebe übereinstimmt.

**Fig. 4** dient der Erläuterung einer weiteren Variante zur Datenreduktion in der erfindungsgemäßen Vorrichtung. Dies soll beispielhaft anhand der Datenreduktion der Daten in der Datenbank 26 mit den Vergleichsdatensätzen 27 erfolgen. Wie bereits oben erläutert kann jeder Ergebnisdatensatz 27 der Datenbank 26 auf einem reduzierten Ergebnisdatensatz abgebildet werden, der im Koordinatensystem aus Durchlaufzeit und Dämpfung als Rechteck repräsentiert wird. Soll nun beispielhaft weiterhin angenommen werden, dass die ersten drei Ergebnisdatensätze 27 in der Datenbank 26 zu Patienten einer bestimmten Eigenschaftsklasse, beispielsweise zu Patienten mit erhöhtem Blutdruck gehören, gehören alle anderen Ergebnisdatensätze in der Datenbank 26 zu Patienten ohne erhöhten Blutdruck. Soll nun für Patienten der Klasse mit erhöhtem Körperblutdruck ein zusätzlich reduzierter Ergebnisdatensatz 34 ermittelt werden, so kann dies geschehen, dass aus den entsprechenden Ergebnisdatensätzen 27 der drei die Klasse repräsentierenden Ergebnisdatensätze, jeweils die zwei Minimal- und Maximalwerte für Durchlaufzeit und Dämpfung ermittelt werden. Diese Klasse von Patienten mit erhöhtem Blutdruck kann dann durch den reduzierten Ergebnisdatensatz 34 repräsentiert werden.

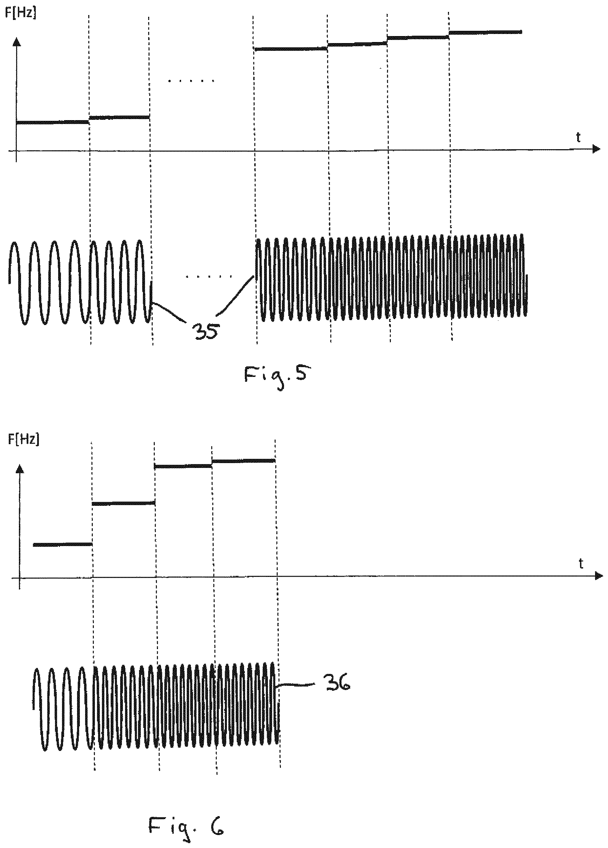

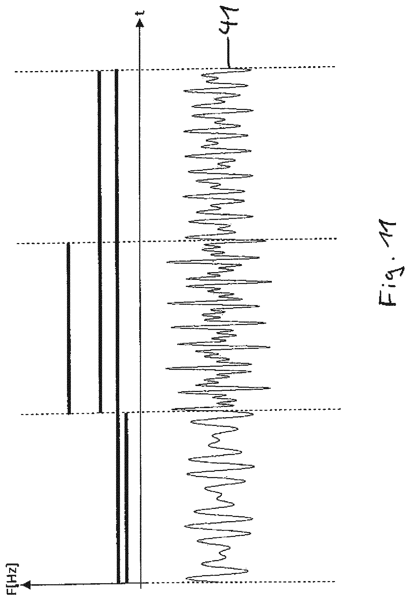

**Fig. 5** bis **Fig. 11** zeigen die Signalverläufe 35 bis 41 in Zeitdomäne, die beispielhaft als Ultraschall-Sendesignale an der Vorrichtung 01 Verwendung finden können. Parallel dazu ist jeweils die Frequenz des Signalverlaufs 35 bis 41 in Hertz angegeben.

Wie bei Signalverlauf 35 kann das Ultraschall-Sendesignal jeweils Abschnitte mit ansteigender Frequenz aufweisen, wobei die Frequenzdifferenz zwischen den einzelnen Abschnitten äquidistant ist.

Wie aus **Fig. 6** ersichtlich, kann die Frequenzdifferenz zwischen den einzelnen Frequenzabschnitten des Signalverlaufs 36 auch nicht äquidistant gewählt werden.

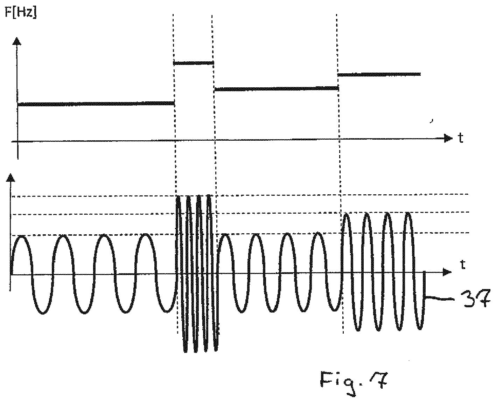

Wie aus **Fig. 7** ersichtlich, kann der Signalverlauf 37 in Abhängigkeit der jeweiligen Sendefrequenz auch im Hinblick auf die Amplitude des Ultraschall-Sendesignals variiert werden.

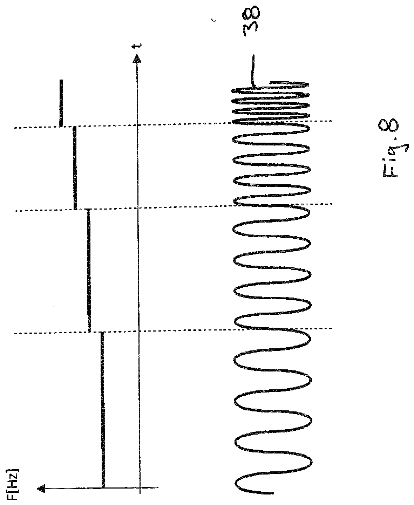

**Fig. 8** stellt einen Signalverlauf 38 des Ultraschall-Sendesignals mit jeweils äquidistant ansteigender Sendefrequenz dar, wobei die Dauer der einzelnen Sendeabschnitte sich zunehmend verkürzt, so dass eine gleichbleibende Anzahl von Durchläufen in jedem Abschnitt vorhanden ist.

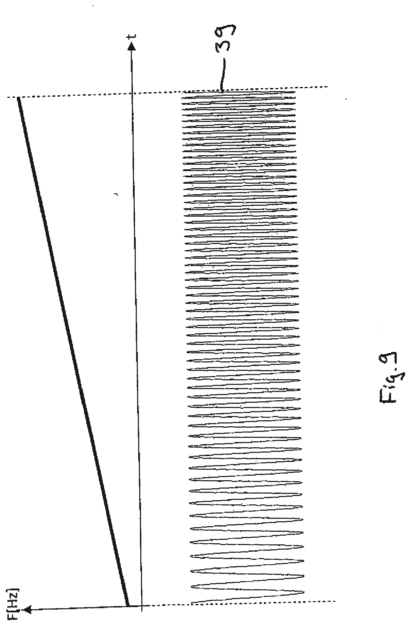

Wie in **Fig. 9** erläutert, kann der Signalverlauf 39 des Ultraschall-Sendesignals auch mit kontinuierlich ansteigender Sendefrequenz, beispielsweise ein linear FM-moduliertes Sendesignal, abgestrahlt werden.

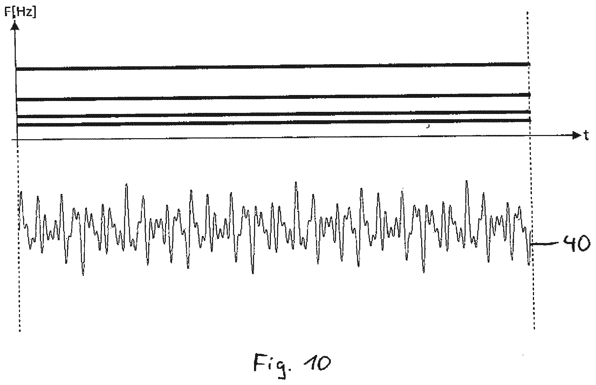

**Fig. 10** stellt einen Sendesignalverlauf 40 mit vier einander überlagerten Sendefrequenzen dar.

Der Signalverlauf 41 des Ultraschall-Sendesignals wird durch Addition verschiedener Sendefrequenzen auf ein gemeinsames Sendesignal (Superposition) erzeugt, wobei einzelne Frequenzen in den verschiedenen Abschnitten hinzukommen oder auch weggelassen werden können.

## Patentansprüche

1. Vorrichtung (01) zur nichtinvasiven Untersuchung eines Körpergewebes (02) durch akustische Spektroskopie, mit einer Ultraschall-Sendeeinrichtung (03) zum Aussenden eines Ultraschall-Sendesignals (35, 36, 37, 38, 39, 40, 41), einer Ultraschall-Empfangseinrichtung (04) zum Empfangen eines reflektierten und/oder transmittierten Ultraschall-Empfangssignals nach Durchlaufen des zu untersuchenden Körpergewebes (02), wobei die Sendeeinrichtung (03) zum Geben mehrerer Ultraschall-Sendesignale unterschiedlicher Frequenzen fi bis fₙ und die Empfangseinrichtung (04) zum Empfangen entsprechender Ultraschall-Empfangssignale unterschiedlicher Frequenz ausgebildet ist,
wobei die Vorrichtung (01) eine erste Verarbeitungseinrichtung (10), eine zweite Verarbeitungseinrichtung (11), eine Signalweiche (09), eine Speichereinrichtung (17) und eine Auswerteeinrichtung (25) umfasst, wobei mit der ersten einen Time-to-digital-Converter (13) umfassenden Verarbeitungseinrichtung (10) die frequenzabhängigen Durchlaufzeitwerte (16) des zugeordneten Ultraschallsignals eines jeden Sende- und Empfangssignalpaares unmittelbar messbar sind, und wobei mit der zweiten Verarbeitungseinrichtung (11) aus jedem Sende- und Empfangssignalpaar die frequenzabhängigen Dämpfungswerte (20) des zugeordneten Ultraschallsignals ermittelbar sind, und wobei zwischen der Ultraschall-Empfangseinrichtung (04) einerseits und den beiden Verarbeitungseinrichtungen (10, 11) andererseits die Signalweiche (09) vorgesehen ist, mit der ein jeweils empfangenes Ultraschall-Empfangssignal identisch zur parallelen Verarbeitung auf beide Verarbeitungseinrichtungen (10, 11) leitbar ist, und wobei in der Speichereinrichtung (17) zu jeder Frequenz fi bis fₙ der verschiedenen Sende- und Empfangssignale, wobei n größer 1 ist, die jeweils ermittelten Durchlaufzeitwerte (16) und Dämpfungswerte (20) zumindest zeitweise als Ergebnisdatensatz (21) abspeicherbar sind, und wobei in der Auswerteeinrichtung (25) aus dem Ergebnisdatensatz (21) ein qualifizierender Wert zur Beschreibung des untersuchten Körpergewebes (02) ableitbar ist.

2. Vorrichtung nach Anspruch 1,
wobei die Vorrichtung (91) eine Datenreduktioneinrichtung (22) umfasst und mit der Datenreduktioneinrichtung (22) aus einem Ergebnisdatensatz (21) oder mehreren Ergebnisdatensätzen (27) ein reduzierter Ergebnisdatensatz (23, 34) abgeleitet werden kann, wobei der reduzierte Ergebnisdatensatz (23, 34) den ermittelten Ergebnisdatensatz (21) oder die verschiedenen Ergebnisdatensätze (27) charakteristisch abbildet und einen geringeren Datenumfang hat.

3. Vorrichtung nach Anspruch 2,
wobei in der Datenreduktioneinrichtung (22) der maximale und der minimale Durchlaufzeitwert in einem ermittelten Ergebnisdatensatz (21) sowie der maximale und der minimale Dämpfungswert in diesem Ergebnisdatensatz (21) bestimmbar ist, wobei diese Maximal- und Minimalwerte als reduzierter Ergebnisdatensatz (23) gespeichert werden.

4. Vorrichtung nach Anspruch 2,
wobei in der Datenreduktioneinrichtung (22) der maximale und der minimale Durchlaufzeitwert von mehreren ermittelten Ergebnisdatensätzen (27), sowie der maximale und der minimale Dämpfungswert in diesen Ergebnisdatensätzen (27) bestimmbar sind, wobei diese Maximal- und Minimalwerte als reduzierter Ergebnisdatensatz (34) gespeichert werden.

5. Vorrichtung nach Anspruch 2,
wobei in der Datenreduktioneinrichtung (22) zumindest ein Quantil der Durchlaufzeitwerte in einem ermittelten Ergebnisdatensatz (21) sowie zumindest ein Quantil der Dämpfungswerte in diesem Ergebnisdatensatz (21) bestimmbar ist, wobei diese Quantile als reduzierter Ergebnisdatensatz (23) gespeichert werden.

6. Vorrichtung nach einem der Ansprüche 2 bis 5,
wobei mit der Auswerteeinrichtung (25) aus dem reduzierten Ergebnisdatensatz (23) zumindest ein das Körpergewebe (02) qualifizierender Wert abgeleitet werden kann.

7. Vorrichtung nach Anspruch 6
wobei die Vorrichtung (01) eine Datenbank (26) umfasst und die Ableitung des qualifizierenden Werts in der Auswerteeinrichtung (25) auf Basis eines Mustervergleichs von ermittelten oder reduzierten Ergebnisdatensätzen (21, 23) mit in der Datenbank (26) gespeicherten Vergleichs-Ergebnisdatensätzen (27, 34) erfolgt, wobei die Vergleichs-Ergebnisdatensätze (27, 34) durch Messung an Vergleichsmaterialien mit bekannten Eigenschaften bestimmt wurden.

8. Vorrichtung nach einem der Ansprüche 1 bis 7
wobei in der ersten Verarbeitungseinrichtung (10) die Durchlaufzeit mit einer Auflösung, insbesondere von zumindest 10 Pikosekunden, ermittelt werden kann.

9. Vorrichtung nach einem der Ansprüche 1 bis 8
wobei die Ultraschall-Sendeeinrichtung (03) und die Ultraschall-Empfangseinrichtung (04) gegenüberliegend an der Außenseite der Kopfhaut eines Patienten temporär und/oder dauerhaft anordenbar sind.

10. Vorrichtung nach einem der Ansprüche 1 bis 9
wobei die Ultraschall-Sendesignale unterschiedlicher Frequenz von der Ultraschall-Sendeeinrichtung (03) einzeln nacheinander ausgesendet werden.

11. Vorrichtung nach einem der Ansprüche 1 bis 10
wobei die Ultraschall-Sendesignale unterschiedlicher Frequenz in der Ultraschall-Sendeeinrichtung (03) auf ein gemeinsames Trägersignal aufmoduliert oder aufaddiert und danach gemeinsam ausgesendet werden.

12. Vorrichtung nach einem der Ansprüche 1 bis 11
wobei die Sendeeinrichtung (03) zum Geben der Ultraschall-Sendesignale mit unterschiedlichen Amplituden ausgebildet ist.

## Claims

1. A device (01) for non-invasively examining body tissue (02) via acoustic spectroscopy, said device (01) comprising an ultrasonic transmission device (03) for transmitting an ultrasonic transmission signal (35, 36, 37, 38, 39, 40, 41), an ultrasonic reception device (04) for receiving a reflected and/or transmitted ultrasonic reception signal after said signal has passed through the body tissue (02) to be examined, said transmission device (03) being configured for giving several ultrasonic transmission signals having different frequencies fi to fₙ and said reception device (04) being configured for receiving corresponding ultrasonic reception signals having different frequencies,
wherein the device (01) comprises a first processing device (10), a second processing device (11), a signal splitter (09), a storage device (17) and an evaluation device (25), wherein the frequency dependent time-of-flight values (16) of the allocated ultrasonic signal of every signal pair consisting of the transmission signal and the reception signal are directly measured using the first processing device (10) that comprises a time-to-digital converter (13),
and wherein the frequency dependent attenuation values (20) of the allocated ultrasonic signal are identified from every signal pair consisting of the transmission signal and the reception signal using the second processing device (11),
and wherein the signal splitter (09) is provided between the ultrasonic reception device (04) on the one hand and the two processing devices (10, 11) on the other hand, a correspondingly received ultrasonic reception signal being able to be directed to both processing devices (10, 11) identically for parallel processing using said signal splitter (09), and wherein for each frequency fi to fₙ of the different transmission signals and reception signals, n being greater than 1, the correspondingly identified time-of-flight values (16) and attenuation values (20) are stored as a results dataset (21) in the storage device (17) at least temporarily, and wherein a qualifying value for describing the examined body tissue (02) is derived from the results dataset (21) in the evaluation device (25).

2. The device according to claim 1,
wherein the device (91) comprises a data reduction device (22) and a reduced results dataset (23, 34) is able to be derived from a results dataset (21) or from several results datasets (27) using the data reduction device (22), said reduced results dataset (23, 34) characteristically depicting the identified results dataset (21) or the several results datasets (27) and having a smaller data volume.

3. The device according to claim 2,
wherein in the data reduction device (22), the maximal time-of-flight value and the minimal time-of-flight value are determined in an identified results dataset (21) and the maximal attenuation value and the minimal attenuation value are determined in said results data set (21), said maximal values and minimal values being stored as a reduced results dataset (23).

4. The device according to claim 2,
wherein in the data reduction device (22), the maximal time-of-flight value and the minimal time-of-flight value are determined from several identified results datasets (27) and the maximal attenuation value and the minimal attenuation value are determined in said results datasets (27), said maximal values and minimal values being stored as a reduced results dataset (34).

5. The device according to claim 2,
wherein in the data reduction device (22), at least one quantile of the time-of-flight values are determined in an identified results dataset (21) and at least one quantile of the attenuation values are determined in said results dataset (21), said quantiles being stored as a reduced results dataset (23).

6. The device according to any one of the claims 2 to 5,
wherein at least one value qualifying the body tissue (02) is able to be derived from the reduced results dataset (23) using the evaluation device (25).

7. The device according to claim 6,
wherein the device (01) comprises a databank (26), and the qualifying value is derived in the evaluation device (25) on the basis of a pattern comparison of identified or reduced results datasets (21, 23) with comparative results datasets (27, 34) stored in the databank (26), said comparative results datasets (27, 34) having been determined by measuring comparative materials having known properties.

8. The device according to any one of the claims 1 to 7,
wherein the time-of-flight is able to be identified with a resolution in particular of at least 10 picoseconds in the first processing device (10).

9. The device according to any one of the claims 1 to 8,
wherein the ultrasonic transmission device (03) and the ultrasonic reception device (04) are temporarily and/or permanently arranged facing each other on the outer side of a patient's scalp.

10. The device according to any one of the claims 1 to 9,
wherein the ultrasonic transmission signals having different frequencies are transmitted individually one after the other by the ultrasonic transmission device (03).

11. The device according to any one of the claims 1 to 10,
wherein the ultrasonic transmission signals having different frequencies are modulated onto or added to a shared carrier signal in the ultrasonic transmission device (03) and are then transmitted together.

12. The device according to any one of the claims 1 to 11,
wherein the transmission device (03) is configured for giving the ultrasonic transmission signals having different amplitudes.

## Revendications

1. Dispositif (01) pour un examen non invasif de tissus corporels (02) par la spectroscopie acoustique, ledit dispositif (01) comprenant un dispositif de transmission (03) ultrasonique pour la transmission d'un signal de transmission (35, 36, 37, 38, 39, 40, 41) ultrasonique, un dispositif de réception (04) ultrasonique pour la réception d'un signal de réception ultrasonique réfléchi et/ou transmis après ledit signal a passé à travers les tissus corporels (02) à être examinés, ledit dispositif de transmission (03) étant configuré pour donner plusieurs signaux de transmission ultrasoniques ayant des fréquences différentes fi à fₙ et ledit dispositif de réception (04) étant configuré pour recevoir des signaux de réception ultrasoniques correspondants ayant des fréquences différentes,
dans lequel le dispositif (01) comprend un premier dispositif de traitement (10), un deuxième dispositif de traitement (11), un duplexeur de signal (09), un dispositif de stockage (17) et un dispositif d'évaluation (25), dans lequel les valeurs de temps de vol (16) du signal ultrasonique assigné de chaque paire de signaux constituée du signal de transmission et du signal de réception sont mesurées directement en utilisant le premier dispositif de traitement (10) qui comprend un convertisseur temps-numérique (13), les valeurs de temps de vol (16) étant dépendantes de la fréquence,
et dans lequel les valeurs d'atténuation (20) du signal ultrasonique assigné sont identifiées à partir de chaque paire de signaux constituée du signal de transmission et du signal de réception en utilisant le deuxième dispositif de traitement (11), les valeurs d'atténuation (20) étant dépendantes de la fréquence, et dans lequel le duplexeur de signal (09) est prévu entre le dispositif de réception (04) ultrasonique d'un côté et les deux dispositifs de traitement (10, 11) d'un autre côté, un signal de réception ultrasonique reçu à chaque fois pouvant être transmis à tous les deux dispositifs de traitement (10, 11) de manière identique pour le traitement parallèle en utilisant ledit duplexeur de signal (09), et dans lequel, pour chaque fréquence fi à fₙ des signaux de transmission différents et des signaux de réception différents, n étant plus grand que 1, les valeurs de temps de vol (16) et les valeurs d'atténuation (20) identifiées à chaque fois sont stockées comme ensemble (21) de données de résultats dans le dispositif de stockage (17) au moins temporairement, et dans lequel une valeur qualifiante pour la description des tissus corporels (02) examinés est dérivée à partir de l'ensemble (21) de données de résultats dans le dispositif d'évaluation (25).

2. Dispositif selon la revendication 1,
dans lequel le dispositif (91) comprend un dispositif (22) de réduction de données et un ensemble (23, 34) de données de résultats réduit peut être dérivé à partir d'un ensemble (21) de données de résultats ou à partir de plusieurs ensembles (27) de données de résultats en utilisant le dispositif (22) de réduction de données, ledit ensemble (23, 34) de données de résultats réduit reproduisant caractéristiquement l'ensemble (21) de données de résultats identifié ou les plusieurs ensembles (27) de données de résultats et ayant un volume de données plus petit.

3. Dispositif selon la revendication 2,
dans lequel, dans le dispositif (22) de réduction de données, la valeur de temps de vol maximale et la valeur de temps de vol minimale sont déterminées dans un ensemble (21) de données de résultats identifié et la valeur d'atténuation maximale et la valeur d'atténuation minimale sont déterminées dans ledit ensemble de données (21) de résultats, lesdites valeurs maximales et lesdites valeurs minimales étant stockées comme ensemble (23) de données de résultats réduit.

4. Dispositif selon la revendication 2,
dans lequel, dans le dispositif (22) de réduction de données, la valeur de temps de vol maximale et la valeur de temps de vol minimale sont déterminées à partir de plusieurs ensembles (27) de données de résultats identifiés et la valeur d'atténuation maximale et la valeur d'atténuation minimale sont déterminées dans lesdits ensembles (27) de données de résultats, lesdites valeurs maximales et lesdites valeurs minimales étant stockées comme ensemble (34) de données de résultats réduit.

5. Dispositif selon la revendication 2,
dans lequel, dans le dispositif (22) de réduction de données, au moins un quantile des valeurs de temps de vol est déterminé dans un ensemble (21) de données de résultats identifié et au moins un quantile des valeurs d'atténuation est déterminé dans ledit ensemble (21) de données de résultats, lesdits quantiles étant stockés comme ensemble (23) de données de résultats réduit.

6. Dispositif selon l'une quelconque des revendications 2 à 5, dans lequel au moins une valeur qualifiant les tissus corporels (02) peut être dérivée à partir de l'ensemble (23) de données de résultats réduit en utilisant le dispositif d'évaluation (25).

7. Dispositif selon la revendication 6,
dans lequel le dispositif (01) comprend une base de données (26), et la valeur qualifiante est dérivée dans le dispositif d'évaluation (25) sur la base d'une comparaison d'échantillon d'ensembles (21, 23) de données de résultats identifiés ou réduits avec des ensembles (27, 34) de données de résultats de référence stockés dans la base de données (26), lesdits ensembles (27, 34) de données de résultats de référence ayant été déterminés par la mesure de matériaux de référence ayant des caractéristiques connues.

8. Dispositif selon l'une quelconque des revendications 1 à 7, dans lequel le temps de vol peut être identifié avec une résolution, notamment d'au moins 10 picosecondes, dans le premier dispositif de traitement (10).

9. Dispositif selon l'une quelconque des revendications 1 à 8, dans lequel le dispositif de transmission (03) ultrasonique et le dispositif de réception (04) ultrasonique sont disposés temporairement et/ou en permanence l'un vis-à-vis de l'autre sur le côté extérieur d'un cuir chevelu d'un(e) patient(e).

10. Dispositif selon l'une quelconque des revendications 1 à 9, dans lequel les signaux de transmission ultrasoniques ayant des fréquences différentes sont transmis individuellement l'un après l'autre par le dispositif de transmission (03) ultrasonique.

11. Dispositif selon l'une quelconque des revendications 1 à 10, dans lequel les signaux de transmission ultrasoniques ayant des fréquences différentes sont modulés sur ou additionnés sur un signal de support commun dans le dispositif de transmission (03) ultrasonique et sont ensuite transmis ensemble.

12. Dispositif selon l'une quelconque des revendications 1 à 11, dans lequel le dispositif de transmission (03) est configuré pour donner des signaux de transmission ultrasoniques ayant des amplitudes différentes.
